(19) Europäisches Patentamt
European Patent Office
Office européen des brevets

(11) **EP 2 029 513 B1**

(12) **FASCICULE DE BREVET EUROPEEN**

(45) Date de publication et mention
de la délivrance du brevet:
**16.08.2017 Bulletin 2017/33**

(21) Numéro de dépôt: **07765951.4**

(22) Date de dépôt: **25.05.2007**

(51) Int Cl.:
*C07C 51/08* *(2006.01)*   *C07C 55/02* *(2006.01)*
*C07C 55/12* *(2006.01)*   *C07C 55/10* *(2006.01)*
*C07C 55/14* *(2006.01)*   *C07C 67/08* *(2006.01)*
*C07C 69/34* *(2006.01)*   *C07C 69/40* *(2006.01)*
*C07C 69/42* *(2006.01)*   *C07C 69/44* *(2006.01)*

(86) Numéro de dépôt international:
**PCT/FR2007/000875**

(87) Numéro de publication internationale:
**WO 2007/141404 (13.12.2007 Gazette 2007/50)**

(54) **PROCEDE DE TRANSFORMATION DE COMPOSES NITRILES EN ACIDES CARBOXYLIQUES ET ESTERS CORRESPONDANTS**

VERFAHREN ZUR UMWANDUNG VON NITRILVERBINDUNGEN IN CARBONSÄUREN UND ENTSPRECHENDE ESTER

PROCESS FOR CONVERTING NITRILE COMPOUNDS TO CARBOXYLIC ACIDS AND CORRESPONDING ESTERS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **09.06.2006 FR 0605119**

(43) Date de publication de la demande:
**04.03.2009 Bulletin 2009/10**

(73) Titulaire: **Rhodia Opérations**
**93306 Aubervilliers (FR)**

(72) Inventeurs:
• **LECONTE, Philippe**
**68150 RIBEAUVILLE (FR)**
• **DENIS, Cyril**
**69003 Lyon (FR)**

(74) Mandataire: **Cardon, Flavie**
**Rhodia Operations**
**Direction Propriété Industrielle**
**40, rue de la Haie-Coq**
**93306 Aubervilliers Cedex (FR)**

(56) Documents cités:
BE-A- 687 139      US-A- 2 388 813
US-A- 3 876 691      US-B1- 6 281 383

• **FALLAHPOUR: "Synthesis of an Isotopically Isomeric Mixture of 1,4,6,8-Tetramethyl[13C2]azulene and Its Thermal Reaction with Dimethyl Acetylendicarboxylate" HELV. CHIM. AKTA, vol. 78, no. 6, 1995, pages 1419-1436, XP009082874**
• **ERNST HANSCHKE: "Zur Kenntnis der Prinsschen Reaktion, II. Mitteil. 1) Über die Umsetzung von Butylen mit Formaldehyd" CHEM. BER., no. 88, 1955, pages 1048-1053, XP009082873**
• **ALLINGER: "The Relative Stabilities of cis and trans Isomers. IV. The 3,5-dimethylcycloheptanones" J AM. CHEM. SOC., vol. 81, 1959, pages 232-235, XP002430861**

## Description

La présente invention concerne un procédé de transformation de composés hydrocarbonés comprenant au moins une fonction nitrile en composés comprenant au moins une fonction carboxylique et également un procédé d'obtention de composés esters à partir de ces composés carboxyliques ainsi obtenus.

L'invention concerne plus particulièrement un procédé d'obtention de composés esters à partir de composés carboxyliques, lesdits composés carboxyliques étant issus de la transformation de composés comprenant au moins une fonction nitrile en composés comprenant au moins une fonction carboxylique et ledit procédé étant caractérisé en ce qu'il consiste à :

- faire réagir un mélange de méthyl-glutaronitrile, éthyl-succinonitrile et adiponitrile issu de la fabrication de l'adiponitrile par hydrocyanation du butadiène avec un composé basique hydroxyle, en présence de solvant à une température comprise entre 80°C et 200°C, conduisant à un mélange de sels des acides méthyl-glutarique, éthyl-succinique et adipique,
- éliminer l'ammoniac formé du milieu réactionnel,
- faire réagir les sels formés des différents acides avec un acide minéral,
- séparer le mélange d'acide méthyl-glutarique, acide éthyl-succinique et acide adipique du milieu réactionnel par extraction liquide/liquide par mise en contact avec un solvant d'extraction polaire non soluble dans l'eau, et
- transformer les composés carboxyliques obtenus en esters par réaction avec un alcool. Le procédé de fabrication d'adiponitrile par hydrocyanation du butadiène est exploité industriellement depuis plusieurs dizaines d'années. Ce procédé présente une sélectivité élevée en adiponitrile, intermédiaire chimique important pour la synthèse de l'hexaméthylène diamine ou du caprolactame et la fabrication des polyamides.

Toutefois, ce procédé produit également des composés dinitriles ramifiés tels que le méthyl-glutaronitrile, l'éthyl-succinonitrile notamment qui sont séparés et récupérés par distillation.

En général, cette récupération des composés dinitriles ramifiés permet de produire un mélange contenant majoritairement du méthyl-glutaronitrile avec de l'éthyl-succinonitrile et de l'adiponitrile.

Plusieurs solutions ont été proposées pour valoriser ces sous-produits ou mélanges. Une de celles-ci consiste à hydrogéner les composés dinitriles en amines primaires notamment pour produire de la méthylpentaméthylènediamine (MPMD), utilisée comme monomère pour la fabrication de polyamides particuliers. Ce procédé requiert des étapes de purification soit du méthyl-glutaronitrile soit de la méthylpentaméthylènediamine.

Dans l'industrie, ces sous-produits sont souvent détruits par combustion avec valorisation sous forme de vapeur ou d'énergie mais avec production d'effluents gazeux contenant du $CO_2$, et des oxydes d'azote.

Le brevet BE687139 divulgue l'hydrolyse basique du 2-méthyl-glutaronitrile en acide 2-méthyl-glutarique. Il existe donc un besoin et une demande importante de trouver de nouvelles voies de valorisation et transformation de ces composés dinitriles ou des mélanges en composés chimiques valorisables et économiquement intéressants.

Un des buts de la présente invention est notamment de proposer un procédé permettant de transformer ces composés nitriles en composés carboxyliques d'une part, utilisables notamment comme intermédiaires chimiques tels que, par exemple, monomères pour la fabrication de polyuréthanes et polyamides, ou en composés diesters d'autre part, utilisables notamment comme solvant.

Selon une caractéristique préférentielle de l'invention, le composé basique hydroxyle est un hydroxyde d'alcalin tel que la soude, la potasse ou analogue. Avantageusement une solution aqueuse de ce composé basique hydroxyle est utilisée. La concentration en composés basiques dans la solution aqueuse est avantageusement comprise entre 5 et 30 % en poids.

Selon une nouvelle caractéristique avantageuse de l'invention, le composé basique hydroxyle est mis en oeuvre avec un excès de 3 à 20%, avantageusement entre 3 et 10% par rapport à la quantité stoechiométrique nécessaire pour transformer les fonctions nitriles en fonction carboxyliques.

La réaction est mise en oeuvre soit sous pression atmosphérique à, avantageusement, une température permettant d'avoir un reflux du solvant, par exemple de l'eau, soit sous pression avec élimination de l'ammoniac formé ou sans élimination de cet ammoniac formé qui se solubilisera au moins partiellement dans le milieu réactionnel.

Dans un mode de réalisation de l'invention, le procédé est mis en oeuvre en continu. La réaction avec le composé alcalin peut être réalisée dans un seul réacteur piston ou dans plusieurs réacteurs agités disposés en série. La réaction est avantageusement réalisée dans un dispositif comprenant deux zones successives et distinctes de réaction, une première zone fonctionnant en régime agité et une seconde zone finale fonctionnant en régime piston. La présence de ces deux zones de réaction permet d'obtenir une transformation totale des fonctions nitriles en fonctions carboxyliques. Cette transformation totale permet de faciliter la récupération et séparation des composés carboxyliques notamment quand les composés nitriles à transformer sont des composés dinitriles et ainsi éviter la présence de composés cyano et/ ou amide dans le milieu réactionnel final. Ainsi, le procédé peut être mis en oeuvre dans un dispositif comprenant un réacteur agité muni d'un condenseur pour obtenir un reflux et un dispositif récupérateur de l'ammoniac formé, et un réacteur piston monté en série avec le réacteur agité. Le réacteur agité peut également fonctionner sous pression sans séparation de l'ammoniac formé avec solubilisation de celui-ci dans le milieu réactionnel.

Le premier réacteur agité peut être avantageusement un réacteur à boucles, comprenant une boucle de circulation interne et une boucle de circulation externe. Un échangeur de chaleur est avantageusement prévu sur la boucle de circulation externe.

Bien entendu, d'autres types de réacteurs agités peuvent être utilisés.

Avantageusement, les températures et les pressions dans le réacteur agité et le réacteur piston peuvent être différentes. Le réacteur piston fonctionne avantageusement de façon adiabatique. De préférence, la température de réaction dans le réacteur piston est plus élevée que celle présente dans le réacteur agité. Ainsi, la température dans le réacteur piston est avantageusement supérieure à 100°C, de préférence comprise entre 100°C et 200°C.

Dans le cas d'un procédé discontinu, les conditions de fonctionnement du réacteur sont déterminées pour obtenir, avantageusement un taux de transformation de 100% des fonctions nitriles.

Le milieu réactionnel en sortie de l'étape de réaction notamment en sortie du réacteur piston contient un composé carboxylate de l'élément métallique introduit avec le composé alcalin, avantageusement un carboxylate de sodium ou de potassium ainsi que de l'ammoniac formé et dissous.

Ce milieu réactionnel est alimenté dans une étape permettant de séparer l'ammoniac formé, par exemple, dans une colonne à distiller ou un flash.

L'ammoniac ainsi récupéré est directement valorisable ou peut être purifié, par exemple par distillation, pour séparer l'eau et obtenir de l'ammoniac valorisable, par exemple, dans les procédés de fabrication d'acide nitrique ou d'acide cyanhydrique.

Selon une caractéristique de l'invention, le milieu réactionnel est ensuite acidifié par addition d'un acide minéral pour obtenir le composé carboxylique sous forme protonée appelé également ci-après, acide carboxylique.

Comme acide minéral convenable pour l'invention, on peut citer l'acide sulfurique, l'acide chlorhydrique, les acides organiques présentant un pKa inférieur ou égal à 3, ou leurs mélanges.

Eventuellement, pour éviter la précipitation des sels, de l'eau peut être ajouté dans le milieu préférentiellement avant l'addition de l'acide minéral. La quantité d'eau ajoutée est déterminée pour obtenir une concentration en sel dans le milieu réactionnel voisine de la concentration saturante du sel dans l'eau.

Cette acidification est mise en oeuvre dans tout type de réacteur, notamment dans des réacteurs agités ou en ligne ou en réacteur tubulaire avec mélangeurs statiques.

La quantité d'acide ajoutée est avantageusement sensiblement égale à la quantité nécessaire pour neutraliser le composé basique ajouté à la première étape.

Le milieu réactionnel est ensuite traité pour récupérer cet acide carboxylique. Le milieu réactionnel peut-être concentré par évaporation de l'eau et le sel est séparé par filtration.

Comme procédé de traitement convenable pour l'invention, l'extraction liquide/liquide de l'acide carboxylique par un solvant non miscible à l'eau est préférée.

Comme solvant d'extraction convenable pour l'invention, on peut citer les solvants polaires non solubles dans l'eau tels que, par exemple, les cétones, les éthers et les esters. On peut citer comme solvant convenable, la méthylisobutylcétone, le diisopropyléther, le méthyltertiobutyléther, les acétates d'éthyle, d'isopropyle, de butyle, les solvants diesters ou analogues.

Les acides carboxyliques sont récupérés dans la phase contenant le solvant d'extraction et sont séparés du solvant avantageusement, par distillation de ce dernier. Les solvants formant un azéotrope hétérogène avec l'eau, entraînant ainsi l'eau contenue dans la phase d'extraction sont préférés. Ainsi, les diacides récupérés en pied de la colonne de séparation du solvant d'extraction ne contiennent pas d'eau ou simplement une très faible quantité d'eau. Le solvant d'extraction est avantageusement recyclé à l'étape d'extraction après séparation de l'eau, par exemple, par décantation.

Selon une autre caractéristique de l'invention, la phase aqueuse récupérée après extraction de l'acide carboxylique et qui contient les sels de l'acide minéral peut être soumise, avant évacuation comme effluent, à un stripping pour récupérer et extraire les traces de solvant d'extraction.

Les acides carboxyliques ainsi obtenus sont, dans un mode de réalisation de l'invention, purifié par les techniques habituelles, à savoir, cristallisation, distillation, raffinage ou analogue.

Ainsi, il est avantageux de soumettre les acides carboxyliques récupérés à une distillation dans une ou plusieurs colonnes pour éliminer d'une part les composés légers et d'autre part les produits lourds. Cette purification est simplifiée si les composés nitriles de départ notamment le méthylglutaronitrile sont purifiés par séparation des composés volatils tels que le crésol et/ou pentènenitriles. Cette purification est également facilitée si la concentration en ammoniac dans les acides à purifier est très faible, limitant ainsi la formation de sels tels que le sulfate d'ammonium.

Préalablement à la purification par distillation, cristallisation ou raffinage des acides carboxyliques, il peut être avantageux de les traiter avec du noir de carbone ou de les soumettre à une hydrogénation ou tout autre traitement de purification usuel. Ces traitements de purification peuvent être mis en oeuvre avec la solution d'acide carboxylique dans le solvant d'extraction.

Après purification, les acides récupérés sont mis en forme pour pouvoir être utilisés dans d'autres procédés. Comme procédé de mise forme, on peut citer l'écaillage, la granulation ou l'extrusion sous forme de jonc. Les composés nitriles sont constitués par les sous produits issus de l'hydrocyanation du butadiène c'est-à-dire, un mélange de méthyl-glutaronitrile, éthyl-succinonitrile et adiponitrile, les acides carboxyliques récupérés sont un mélange d'acide méthyl glutarique, acide éthyl-succinique et acide adipique. De tels diacides peuvent trouver de nombreuses applications notamment comme monomères ou comonomères dans la fabrication de polyamides, polyesters et polyuréthanes ou comme additifs.

Selon l'invention, les acides carboxyliques récupérés après séparation du solvant d'extraction sont utilisés pour la fabrication de diesters. Ces diesters seront appelés de manière générique, dans le présent texte "diesters".

Pour la fabrication de ces diesters, les diacides récupérés, avant ou après purification, sont mélangés avec un alcool. L'estérification est réalisée par chauffage du mélange en présence d'un composé acide pour catalyser la réaction.

Ainsi, cette réaction peut être réalisée par passage sur une résine sulfonique acide à une température comprise entre 40 et 100°C. Le milieu issu de la colonne contenant la résine est ensuite distillé pour séparer l'alcool n'ayant pas réagi et l'eau formé.

Elle peut également être mise en oeuvre dans une colonne à distiller réactive permettant de réaliser la réaction d'estérification simultanément à l'extraction de l'eau formée par distillation. Ainsi, il est possible de déplacer l'équilibre pour obtenir un taux de transformation voisin de 100%.

Les alcools convenables sont, par exemple, les alcools aliphatiques ramifiés ou non, cycliques ou non, pouvant comporter un noyau aromatique et pouvant comprendre de 1 à 20 atomes de carbone. A titre d'exemples préférés, on peut citer les alcools suivants, méthanol, propanol, isopropanol, alcool benzylique, éthanol, n-butanol, isobutanol, cyclohexanol, hexanol, isooctanol, 2-éthyl hexanol, pentanols, méthyl-butanol ou analogues.

Le milieu contenant les diesters est ensuite, avantageusement, distillé dans une installation comprenant une étape d'étêtage et d'équeutage. Cette distillation peut être réalisée dans une seule colonne avec récupération des diesters sous forme d'une fraction intermédiaire.

Avantageusement, la fraction lourde récupérée comprend les composés monoester qui sont au moins partiellement récupérés et recyclés dans l'étape d'estérification. Les diesters ainsi obtenus peuvent être utilisés dans de nombreuses applications telles que comme solvant dans les peintures, vernis, laques, l'industrie du revêtement de surfaces ou tout autre articles comme les câbles par exemple, l'industrie des encres des lubrifiants pour textiles, les liants et résines pour noyaux de moules de fonderie, les produits nettoyants, les formulations cosmétiques. Ils peuvent également être utilisés comme matières premières pour certaines réactions chimiques, dans les compositions de traitement des sols et végétaux.

Plus généralement, ils peuvent être utilisés seul ou dans une formulation, comme solvant de nettoyage, décapage, dégraissage dans toute activité industrielle ou domestique.

Ces diesters peuvent également être utilisés comme plastifiants de certaines matières plastiques ou comme monomères pour la fabrication de polymères.

D'autres avantages, détails de l'invention apparaîtront plus clairement au vu des exemples donnés ci-dessous uniquement à titre indicatif.

Hydrolyse basique de composés dinitriles :

Sur 560 g d'une solution aqueuse de soude à 15%, préalablement chauffée à 80°C, 108 g d'un mélange de composés dinitriles provenant de la fabrication d'adiponitrile par hydrocyanation du butadiène sont ajoutés sous agitation en 30 min.

Le mélange de dinitriles a la composition pondérale suivante :

| | |
|---|---|
| Méthyl-glutaronitrile (MGN) : | 84,2 % |
| Ethyl-succinonitrile (ESN) : | 11 % |
| Adiponitrile (AdN) : | 4 % |

Le complément à 100% correspond à différentes impuretés ne comprenant pas des fonctions nitriles.

Le mélange est ensuite chauffé pour obtenir un reflux et est maintenu à cette température pendant 7 h environ.

L'ammoniac qui se dégage est récupéré et piégé.

L'avancement de la réaction est contrôlé par dosage potentiométrique avec une solution d'acide chlorhydrique. Ce dosage permet de déterminer la quantité de soude restante, la quantité d'ammoniac dissous et la quantité de fonction carboxylique salifiée correspondant aux différents sauts de pH observés au cours du dosage.

Le rendement de l'hydrolyse est donné par la formule suivante :

$$RR\% = V_{COONa} \times 100 / (V_{NaOH} + V_{COONa}) \times NaOH\ i\ /\ MGN\ i$$

dans laquelle :

$V_{COONa}$ : correspond au volume de solution de HCl nécessaire pour neutraliser les fonctions carboxyliques salifiées,
$V_{NaOH}$ correspond au volume de solution de HCl nécessaire pour neutraliser la soude résiduelle
NaOH i correspond au nombre de moles de NaOH initial
MGN i correspond au nombre de moles de composés dinitriles initial
RR est le taux de transformation des composés dinitriles en acide carboxylique.

On considère que la réaction est terminée lorsque RR % est proche de 100%.
Le milieu réactionnel est ensuite refroidi à l'ambiante et 80 g d'eau sont additionnées avant de couler 105 g d'acide sulfurique 98%. Le pH de la solution aqueuse est de l'ordre de 3.
La phase aqueuse est ensuite extraite avec 3 fois 200 ml de MTBE (méthyltertiobutyléther) à 40°C. Les phases organiques sont regroupées et le MTBE est ensuite distillé. 141,6 g de diacides sont obtenus avec une pureté déterminée par dosage potentiométrique de l'ordre de 98,5%.

Estérification.

Les 141,6 g de diacides bruts sont solubilisés dans 621 g de méthanol. Cette solution est ensuite placée dans un ballon contenant 115 g de résine sulfonique
(commercialisée par Rhom et Haas sous l'appellation Amberlyst 36 DRY)
préalablement lavée au méthanol.
On porte l'ensemble à ébullition pendant 6 h.
Le dosage des fonctions acides est réalisé par potentiométrie avec de la soude montrant que le taux de transformation atteint 95%.
Après refroidissement, les résines sont séparées par filtration et lavées 3 fois par 150 ml de méthanol. Les différentes phases méthanoliques sont regroupées.
Le méthanol et l'eau formée lors de l'estérification sont séparés par distillation.
Les diesters bruts sont ensuite distillés à 105°C sous 20 mbar sur une colonne à remplissage Un rendement en diesters de 93% est obtenu avec une pureté déterminée par analyse chromatographique supérieure à 99%.

**Revendications**

1. Procédé d'obtention de composés esters à partir de composés carboxyliques, lesdits composés carboxyliques étant issus de la transformation de composés comprenant au moins une fonction nitrile en composés comprenant au moins une fonction carboxylique et ledit procédé étant **caractérisé en ce qu'**il consiste à :

   - faire réagir un mélange de méthyl-glutaronitrile (MGN), éthyl-succinonitrile (ESN) et adiponitrile (ADN) issu de la fabrication de l'adiponitrile par hydrocyanation du butadiène avec un composé basique hydroxyle, en présence de solvant à une température comprise entre 80°C et 200°C, conduisant à un mélange de sels des acides méthyl-glutarique, éthyl-succinique et adipique,
   - éliminer l'ammoniac formé du milieu réactionnel,
   - faire réagir les sels formés des différents acides avec un acide minéral,
   - séparer le mélange d'acide méthyl-glutarique, acide éthyl-succinique et acide adipique du milieu réactionnel par extraction liquide/liquide par mise en contact avec un solvant d'extraction polaire non soluble dans l'eau, et
   - transformer les composés carboxyliques obtenus en esters par réaction avec un alcool.

2. Procédé selon la revendication 1, **caractérisé en ce que** le composé basique hydroxyle est choisi dans le groupe comprenant la soude, la potasse.

3. Procédé selon l'une des revendications précédentes, **caractérisé en ce que** le composé basique hydroxyle précité est utilisé sous forme de solution aqueuse, de préférence avec une concentration pondérale en composé basique comprise entre 5 et 30%.

4. Procédé selon l'une des revendications précédentes, **caractérisé en ce que** la quantité de composé basique hydroxyle mise en oeuvre représente 103 à 120% de la quantité stoechiométrique de composé basique calculée par rapport à la quantité de mélange de MGN, ESN et ADN à transformer.

5. Procédé selon l'une des revendications précédentes, **caractérisé en ce que** la réaction entre le mélange de MGN, ESN et ADN et le composé basique hydroxyle est réalisée à reflux du solvant ou sous pression.

6. Procédé selon l'une des revendications précédentes, **caractérisé en ce que** la réaction entre le mélange de MGN, ESN et ADN et le composé basique hydroxyle est réalisée en discontinu.

7. Procédé selon l'une des revendications 1 à 5, **caractérisé en ce que** la réaction entre le mélange de MGN, ESN et ADN et le composé basique hydroxyle est réalisée en continu.

8. Procédé selon la revendication 7, **caractérisé en ce que** ladite réaction est réalisée dans un dispositif comprenant deux zones de réaction successives et distinctes :

   - une première zone fonctionnant selon un régime agité
   - une seconde zone fonctionnant selon un régime piston

9. Procédé selon l'une des revendications précédentes, **caractérisé en ce que** la quantité d'acide minéral utilisée est au moins égale à la quantité stoechiométrique calculée pour neutraliser le composé basique hydroxyle ajouté.

10. Procédé selon l'une des revendications précédentes, **caractérisé en ce que** l'acide minéral est choisi dans le groupe comprenant l'acide chlorhydrique, l'acide sulfurique, les acides organiques présentant un pKa inférieur ou égal à 3 ou leurs mélanges.

11. Procédé selon l'une des revendications précédentes, **caractérisé en ce que** l'on évite la précipitation de sels par ajout d'eau dans le milieu avant addition de l'acide minéral.

12. Procédé selon la revendication 1, **caractérisé en ce que** le solvant d'extraction est choisi dans le groupe comprenant les cétones, les éthers et les esters.

13. Procédé selon la revendication 12, **caractérisé en ce que** le solvant d'extraction est choisi dans le groupe comprenant la méthylisobutylcétone, le diisopropyléther, le méthyltertiobutyléther, les acétates d'éthyle, d'isopropyle, de butyle et les solvants diesters.

14. Procédé selon l'une des revendications précédentes, **caractérisé en ce que** les composés carboxyliques extraits sont séparés du solvant d'extraction par distillation.

15. Procédé selon l'une des revendications précédentes, **caractérisé en ce que** les composés carboxyliques récupérés sont purifiés par distillation, cristallisation, raffinage.

16. Procédé selon l'une des revendications précédentes, **caractérisé en ce que** l'alcool est choisi dans le groupe comprenant les alcools aliphatiques ramifiés ou non, cycliques ou non, pouvant comporter un noyau aromatique et pouvant comprendre de 1 à 20 atomes de carbone.

17. Procédé selon l'une quelconque des revendications précédentes, **caractérisé en ce que** l'alcool est choisi dans le groupe comprenant méthanol, propanol, isopropanol, alcool benzylique, éthanol, n- butanol, isobutanol, cyclohexanol, hexanol, isooctanol, 2-éthyl hexanol, les isomères des pentanols, l'isobutanol.

18. Procédé selon l'une quelconque des revendications précédentes, **caractérisé en ce que** la réaction d'estérification est réalisée en présence d'ions $H^+$.

19. Procédé selon l'une quelconque des revendications précédentes, **caractérisé en ce que** la réaction d'estérification est réalisée dans une colonne réactive avec élimination de l'eau.

20. Procédé selon l'une quelconque des revendications précédentes, **caractérisé en ce que** la réaction d'estérification est réalisée par passage du mélange composé carboxylique/alcool sur une résine sulfonique acide échangeuse d'ions.

21. Procédé selon l'une quelconque des revendications précédentes, **caractérisé en ce que** les esters formés sont récupérés et purifiés par distillation.

**Patentansprüche**

1. Verfahren zur Gewinnung von Esterverbindungen aus Carbonsäureverbindungen, wobei die Carbonsäureverbindungen aus der Umwandlung von Verbindungen, die mindestens eine Nitrilfunktion umfassen, in Verbindungen, die mindestens eine Carbonsäurefunktion umfassen, stammen und das Verfahren **dadurch gekennzeichnet ist, dass** man :

   - ein Gemisch von Methylglutaronitril (MGN), Ethylsuccinonitril (ESN) und Adiponitril (ADN), das aus der Herstellung von Adiponitril durch Hydrocyanierung von Butadien stammt, mit einer basischen Hydroxylverbindung in Gegenwart eines Lösungsmittels bei einer Temperatur zwischen 80°C und 200°C umsetzt, was zu einem Gemisch von Salzen von Methylglutar-, Ethylbernstein- und Adipinsäure führt,
   - den gebildeten Ammoniak aus dem Reaktionsmedium entfernt,
   - die gebildeten Salze der verschiedenen Säuren mit einer Mineralsäure umsetzt,
   - das Gemisch von Methylglutarsäure, Ethylbernsteinsäure und Adipinsäure vom Reaktionsmedium durch Flüssig-Flüssig-Extraktion mittels Inkontaktbringen mit einem polaren, nicht wasserlöslichen Extraktionslösungsmittel abtrennt und
   - die erhaltenen Carbonsäureverbindungen durch Umsetzung mit einem Alkohol in Ester umwandelt.

2. Verfahren nach Anspruch 1, **dadurch gekennzeichnet, dass** die basische Hydroxylverbindung aus der Natronlauge, Kalilauge umfassenden Gruppe ausgewählt ist.

3. Verfahren nach einem der vorhergehenden Ansprüche, **dadurch gekennzeichnet, dass** die vorstehend genannte basische Hydroxylverbindung in Form einer wässrigen Lösung, vorzugsweise mit einer Konzentration an basischer Verbindung zwischen 5 und 30 %, bezogen auf Gewicht, verwendet wird.

4. Verfahren nach einem der vorhergehenden Ansprüche, **dadurch gekennzeichnet, dass** die eingesetzte Menge an basischer Hydroxylverbindung 103 bis 120 % der stöchiometrischen Menge an basischer Verbindung, berechnet bezogen auf die Menge des Gemischs von umzuwandelndem MGN, ESN und ADN, darstellt.

5. Verfahren nach einem der vorhergehenden Ansprüche, **dadurch gekennzeichnet, dass** die Umsetzung zwischen dem Gemisch von MGN, ESN und ADN und der basischen Hydroxylverbindung unter Rückfluss des Lösungsmittels oder unter Druck durchgeführt wird.

6. Verfahren nach einem der vorhergehenden Ansprüche, **dadurch gekennzeichnet, dass** die Umsetzung zwischen dem Gemisch von MGN, ESN und ADN und der basischen Hydroxylverbindung diskontinuierlich durchgeführt wird.

7. Verfahren nach einem der Ansprüche 1 bis 5, **dadurch gekennzeichnet, dass** die Umsetzung zwischen dem Gemisch von MGN, ESN und ADN und der basischen Hydroxylverbindung kontinuierlich durchgeführt wird.

8. Verfahren nach Anspruch 7, **dadurch gekennzeichnet, dass** die Umsetzung in einer Vorrichtung durchgeführt wird, die zwei aufeinander folgende und getrennte Reaktionszonen umfasst :

   - eine erste Zone, die gemäß einem Verwirbelungsprotokoll arbeitet,
   - eine zweite Zone, die gemäß einem Druckkolbenprotokoll arbeitet.

9. Verfahren nach einem der vorhergehenden Ansprüche, **dadurch gekennzeichnet, dass** die verwendete Menge an Mineralsäure mindestens gleich der stöchiometrischen Menge ist, die für die Neutralisation der hinzugefügten basischen Hydroxylverbindung berechnet worden ist.

10. Verfahren nach einem der vorhergehenden Ansprüche, **dadurch gekennzeichnet, dass** die Mineralsäure aus der Gruppe ausgewählt ist, die Salzsäure, Schwefelsäure, organische Säuren mit einem pKa-Wert von weniger als oder gleich 3 und deren Gemische umfasst.

11. Verfahren nach einem der vorhergehenden Ansprüche, **dadurch gekennzeichnet, dass** man die Ausfällung von Salzen durch Zugabe von Wasser in das Medium vor der Zugabe der Mineralsäure verhindert.

12. Verfahren nach Anspruch 1, **dadurch gekennzeichnet, dass** das Extraktionslösungsmittel aus der Gruppe ausgewählt ist, die Ketone, Ether und Ester umfasst.

13. Verfahren nach Anspruch 12, **dadurch gekennzeichnet, dass** das Extraktionslösungsmittel aus der Gruppe ausgewählt ist, die Methylisobutylketon, Diisopropylether, Methyl-tert-butylether, Ethyl-, Isopropyl-, Butylacetat und Diester-Lösungsmittel umfasst.

14. Verfahren nach einem der vorhergehenden Ansprüche, **dadurch gekennzeichnet, dass** die extrahierten Carbonsäureverbindungen durch Destillation von dem Extraktionslösungsmittel abgetrennt werden.

15. Verfahren nach einem der vorhergehenden Ansprüche, **dadurch gekennzeichnet, dass** die gewonnenen Carbonsäureverbindungen durch Destillation, Kristallisation, Raffination gereinigt werden.

16. Verfahren nach einem der vorhergehenden Ansprüche, **dadurch gekennzeichnet, dass** der Alkohol aus der Gruppe ausgewählt ist, die verzweigte oder nicht verzweigte, zyklische oder nicht zyklische aliphatische Alkohole umfasst, die einen aromatischen Kern enthalten können und 1 bis 20 Kohlenstoffatome umfassen können.

17. Verfahren nach einem der vorhergehenden Ansprüche, **dadurch gekennzeichnet, dass** der Alkohol aus der Gruppe ausgewählt ist, die Methanol, Propanol, Isopropanol, Benzylalkohol, Ethanol, n-Butanol, Isobutanol, Cyclohexanol, Hexanol, Isooctanol, 2-Ethylhexanol, Isomere von Pentanolen, Isobutanol umfasst.

18. Verfahren nach einem der vorhergehenden Ansprüche, **dadurch gekennzeichnet, dass** die Veresterungsreaktion in Gegenwart von $H^+$-Ionen durchgeführt wird.

19. Verfahren nach einem der vorhergehenden Ansprüche, **dadurch gekennzeichnet, dass** die Veresterungsreaktion in einer reaktiven Kolonne unter Entfernung von Wasser durchgeführt wird.

20. Verfahren nach einem der vorhergehenden Ansprüche, **dadurch gekennzeichnet, dass** die Veresterungsreaktion mittels Leiten des Carbonsäureverbindung/Alkohol-Gemischs über ein Sulfonsäure-Ionenaustauscherharz durchgeführt wird.

21. Verfahren nach einem der vorhergehenden Ansprüche, **dadurch gekennzeichnet, dass** die gebildeten Ester mittels Destillation gewonnen und gereinigt werden.

**Claims**

1. Process for obtaining ester compounds from carboxyl compounds, said carboxyl compounds resulting from the conversion of compounds comprising at least one nitrile functional group to give compounds comprising at least one carboxyl functional group and said process being **characterized in that** it consists in :

- reacting a mixture of methylglutaronitrile (MGN), ethylsuccinonitrile (ESN) and adiponitrile (ADN) resulting from the manufacture of adiponitrile by hydrocyanation of butadiene with a basic hydroxyl compound in the presence of a solvent at a temperature of between 80°C and 200°C, resulting in a mixture of salts of methylglutaric, ethylsuccinic and adipic acids,
- removing the ammonia formed from the reaction medium,
- reacting the salts formed of the different acids with an inorganic acid,
- separating the mixture of methylglutaric acid, ethylsuccinic acid and adipic acid from the reaction medium by liquid/liquid extraction by bringing into contact with a water-insoluble polar extraction solvent, and
- converting the carboxyl compounds obtained into esters by reaction with an alcohol.

2. Process according to Claim 1, **characterized in that** the basic hydroxyl compound is chosen from the group consisting of sodium hydroxide and potassium hydroxide.

3. Process according to either of the preceding claims, **characterized in that** the abovementioned basic hydroxyl compound is used in the form of an aqueous solution, preferably with a concentration by weight of basic compound of between 5 and 30 %.

4. Process according to one of the preceding claims, **characterized in that** the amount of basic hydroxyl compound employed represents from 103 to 120 % of the stoichiometric amount of basic compound, calculated with respect to the amount of MGN, ESN and ADN mixture to be converted.

5. Process according to one of the preceding claims, **characterized in that** the reaction between the MGN, ESN and ADN mixture and the basic hydroxyl compound is carried out at reflux of the solvent or under pressure.

6. Process according to one of the preceding claims, **characterized in that** the reaction between the MGN, ESN and ADN mixture and the basic hydroxyl compound is carried out batchwise.

7. Process according to one of Claims 1 to 5, **characterized in that** the reaction between the MGN, ESN and ADN mixture and the basic hydroxyl compound is carried out continuously.

8. Process according to Claim 7, **characterized in that** said reaction is carried out in a device comprising two successive and separate reaction regions :

   - a first region operating according to stirred conditions
   - a second region operating according to plug-flow conditions.

9. Process according to one of the preceding claims, **characterized in that** the amount of inorganic acid used is at least equal to the stoichiometric amount calculated in order to neutralize the basic hydroxyl compound added.

10. Process according to one of the preceding claims, **characterized in that** the inorganic acid is chosen from the group consisting of hydrochloric acid, sulfuric acid, organic acids exhibiting a pKa of less than or equal to 3, and their mixtures.

11. Process according to one of the preceding claims, **characterized in that** the precipitation of salts is avoided by addition of water to the medium before addition of the inorganic acid.

12. Process according to Claim 1, **characterized in that** the extraction solvent is chosen from the group consisting of ketones, ethers and esters.

13. Process according to Claim 12, **characterized in that** the extraction solvent is chosen from the group consisting of methyl isobutyl ketone, diisopropyl ether, methyl tert-butyl ether, ethyl acetate, isopropyl acetate, butyl acetate and diester solvents.

14. Process according to one of the preceding claims, **characterized in that** the carboxylic compounds extracted are separated from the extraction solvent by distillation.

15. Process according to one of the preceding claims, **characterized in that** the carboxylic compounds recovered are purified by distillation, crystallization or refining.

16. Process according to one of the preceding claims, **characterized in that** the alcohol is chosen from the group consisting of linear or branched and cyclic or noncyclic aliphatic alcohols which can comprise an aromatic nucleus and which can comprise from 1 to 20 carbon atoms.

17. Process according to any one of the preceding claims, **characterized in that** the alcohol is chosen from the group consisting of methanol, propanol, isopropanol, benzyl alcohol, ethanol, n-butanol, isobutanol, cyclohexanol, hexanol, isooctanol, 2-ethylhexanol, pentanol isomers and isobutanol.

18. Process according to any one of the preceding claims, **characterized in that** the esterification reaction is carried out in the presence of $H^+$ ions.

19. Process according to any one of the preceding claims, **characterized in that** the esterification reaction is carried out in a reactive column with removal of the water.

20. Process according to any one of the preceding claims, **characterized in that** the esterification reaction is carried out by passing the carboxylic compound/alcohol mixture over a sulfonic acid ion-exchange resin.

21. Process according to any one of the preceding claims, **characterized in that** the esters formed are recovered and purified by distillation.

**RÉFÉRENCES CITÉES DANS LA DESCRIPTION**

**Documents brevets cités dans la description**

* BE 687139